# EUROPEAN PATENT SPECIFICATION

(11) **EP 3 781 876 B1**
(45) Date of publication and mention of the grant of the patent: **03.01.2024**
(21) Application number: 18750257.0
(22) Date of filing: 14.04.2018
(51) Int. Cl.: F24F 8/30, F28D 20/00, F24F 5/00, A61L 9/22

(54) **DEVICE AND METHOD FOR AIR-ION PURIFICATION OF BUILDING INDOOR CLIMATE**
VORRICHTUNG UND VERFAHREN ZUR REINIGUNG DES GEBÄUDEINNENKLIMAS MIT LUFT-IONEN
DISPOSITIF ET PROCÉDÉ DE PURIFICATION PAR AÉRO ION D'UN CLIMAT INTÉRIEUR DE BÂTIMENT

(43) Date of publication of application: 24.02.2021
(73) Proprietor: OÜ Jundap Holding, 12915 Tallinn (EE)
(72) Inventor: JÜRIS, Heiki, 12915 Tallinn (EE)
(74) Representative: Renaudo, Adrien Hanouar
(86) International application number: PCT/IB2018/000578
(87) International publication number: WO 2019/197859

(56) References cited:
- EP-A1- 0 777 088
- WO-A1-2009/006908
- WO-A1-2009/050795

## Description

### TECHNICAL FIELD

The present invention relates to a method and device for treating the natural air blown into rooms, in particular a geothermal method for ion purification of the air with a system for the purification of natural air from pollutants, and for preheating or for cooling.

### BACKGROUND ART

Today, increasingly strict requirements are set for building ventilation systems. Particularly, there is a need for clean and ionized air in cities, because the outside air is no longer of high quality. Air ionizers are used to keep the indoor electro-climate balanced. Conventional air conditioners, ventilation systems and home appliances reduce the amount of negative air ions in the house. The air blown from the ventilation system into the building is clean but contains only positive air ions. Negative ions do not reach indoors because the lifespan of negative air ions is very short. To increase the number of negative air ions, air ionizers are used, which produce negative air ions. As an additional effect, negative air ions collect the dust flying in the air, which is especially important for people who are suffering from allergy problems. Also, negative air ions destroy the bacteria flying in the air.

The well-known Russian scientist Aleksander Tchizhevsky (1879-1964) studied aeroionic hunger in his laboratories and discovered that the amount of negative aeroions in the air is very important. In his experiments, the scientist enriched filtered air with negative aeroions using an electrostatic ionizer. During the experiments, the mice felt significantly better when breathing in the air enriched with negative aeroions and their lifespan was also increased. When the air entry was blocked, the mice soon became lethargic and were barely breathing. However, they started moving, sniffing the air and began to walk around in the test chamber as soon as the ionizer was switched on. The former "lifeless" situation was restored when the ionizer was switched off. Tests have shown that a variety of diseases can be treated by ionizing the air; it has become clear that the so-called aeroionic hunger is a reason for the decrease in the resistance power of the human body, which means that the air that people breathe in is to a great extent lacking negative aeroions. For example, in urban apartments, the number of negative aeroions is 50-100 ions/cm³, on city streets it is 100-500 ions/cm³, in the forest and seaside it is 1,000-5,000 ions/cm³, in mountain resorts it is 5000-10000 ions/cm³, after a thunderstorm it is 50,000-100,000 ions/cm³. The amount of negative ions necessary for life is 3,000-5,000 ions/cm³ and with over 20,000 ions/cm³, a therapeutic effect on the human body begins. For the purpose of aeroions treatment, the researcher designed a device that resembled a chandelier - a circular metal mesh with a diameter of about 1 m, hung on insulators and connected to a high voltage source (100 kV), with needles soldered on the network intersections, from which negative aeroions dropped as a result of corona discharge.

As a result, various electric air ionizers have been developed in prior art to generate negative aeroions. A radioisotope aeroion generator is described in RU545226, 30.10.1980. Further patent literature anticipating the use of geothermal energy to preheat and purify fresh air using negatively charged ions is disclosed in WO 2009/006908 A1.

### DISCLOSURE OF THE INVENTION

The object of the present invention is to provide a purifying system for indoor climate and for the air blown into buildings, which uses ion purification of air for cleaning. The ion purification is based on the natural phenomenon in which airborne particles with different charges (aeroions) try to move away from each other, while, for example, positively charged air particles (positive aeroions) tend to move towards positively charged materials, or, a positive particle joins a positive particle. By utilizing this property, it is possible to collect positive air ions and guide them out from the building and its ventilation system, using the method and purification system proposed in the present invention. In this way, the air that is blown into the building, is enriched with negatively charged aeroions, which in turn means that fresher air is blown into the building. The peculiarity of the system is the use of earth negative potential and the use of earth geothermal energy to heat or cool the air blown into the building.

The objects of the invention are achieved by means of a device as defined in claim 1, comprising a vertical shaft fully or partially placed into the ground, in which are formed various pressure relief chambers, pipelines for guiding the ambient air into the device, and for guiding the outlet air from the building into the device, whereas the air leaving the building is dominated by positive aeroions, which are collected by the ventilation duct and guided out of the device, the air blown into the building is at the same time enriched with negative aeroions. To separate and collect positive aeroions, a ceramsite element that surrounds the ventilation pipe has been placed in the first pressure relief chamber. The positively charged ceramsite attracts positive airborne aeroions and by the speed of moving air and due to the resulting underpressures, positive aeroions are directed to the ventilation duct and, from there, out of the device.

### BRIEF DESCRIPTION OF DRAWINGS

Current invention is described in more details in the following exemplary embodiments with references to annexed figures where:
Fig.1 shows a sectional view of the ion purification device according to the invention;

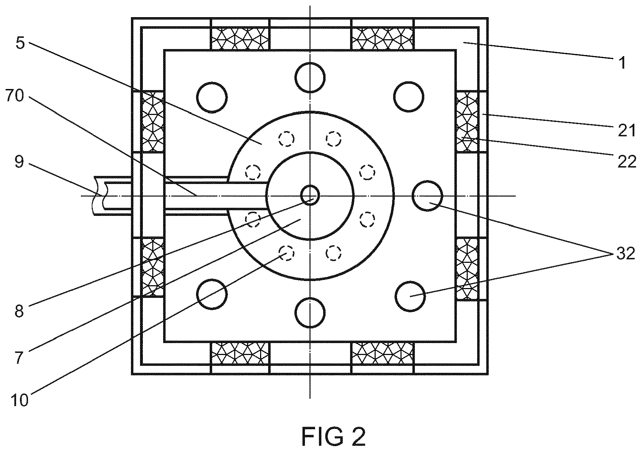

Fig. 2 is a sectional view of the ion purification system along the line D-D on Fig. 1, to illustrate the design of the ambient air intake chamber;

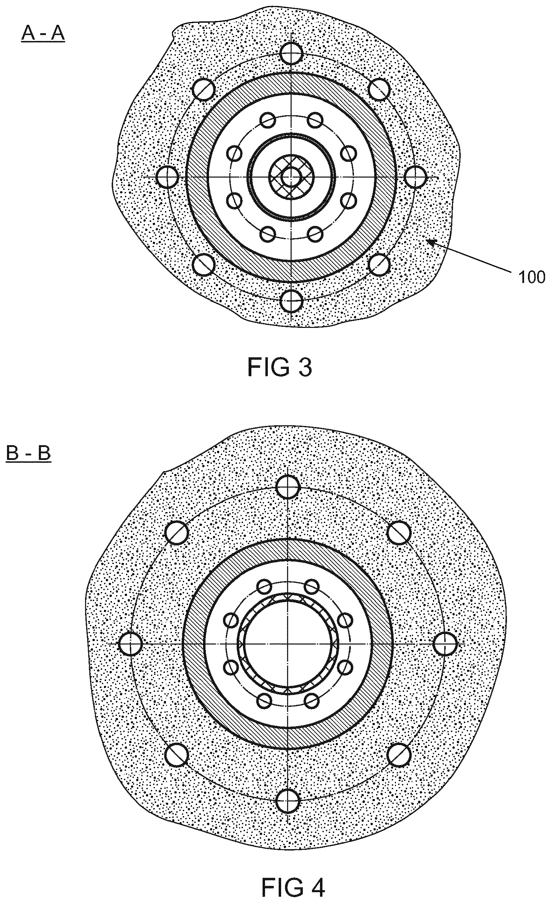

Fig. 3 is a sectional view of the ion purification device along the line A-A on Fig. 1;

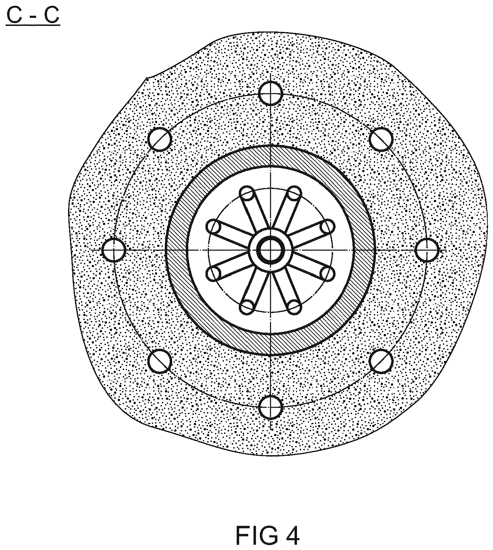

Fig. 4 is a sectional view of the ion purification device along the line B-B on Fig. 1;
Fig. 5 is a sectional view of the ion purification device along the line C-C on Fig. 1;

### DETAILED DESCRIPTION OF INVENTION

A geothermal device/system for ion purification of the indoor air of buildings consists of a housing (1) installed in the ground. In a first embodiment of the invention, part of the housing of the ion purification geothermal device is placed into the ground and part of the housing remains on the ground. However, the entire device can be installed into the ground and the air is fed into the device through the pipes. The housing (1) can be a cast concrete shaft having a base with cylindrical, rectangular (e.g. a square) or polygonal cross-section. Hence, the shaft may be prefabricated in a factory and delivered to the installation site as a single structure, which is then partly dug into the ground and partly left on top of the ground.

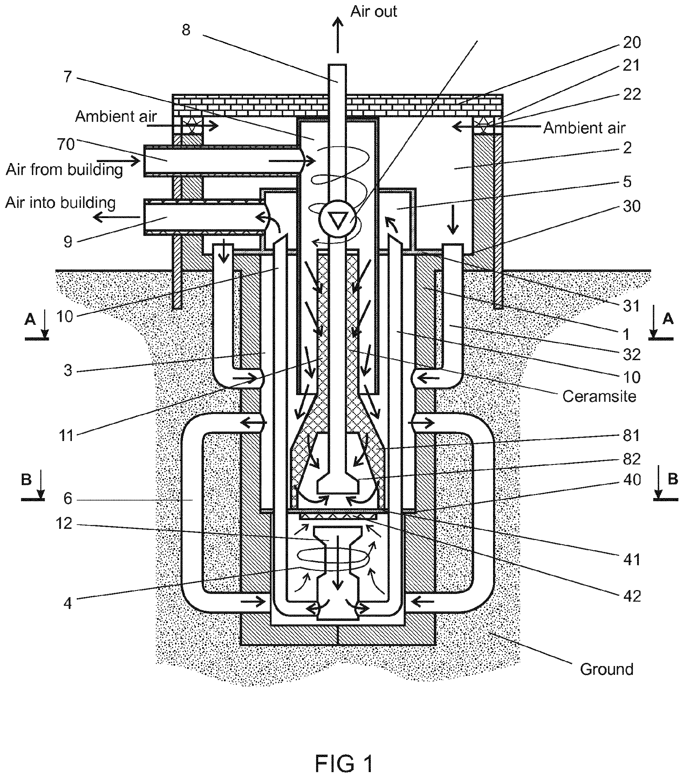

The structure of the shaft includes three parts: the upper part above the ground, or the air intake chamber (2), the middle part below the ground level, or the first pressure relief chamber (3), and the lower part, also below the ground level, or the second pressure relief chamber (4). In this case, the dimensions of the first pressure relief chamber (e.g. for a cylindrical housing, the diameter) are larger than the dimensions of the other pressure chamber, so that a step (40) is formed at the transition between the first and second pressure relief chambers, to which is placed and secured a lid (41), which separates the second pressure relief chamber (4) from the first pressure relief chamber (3), and through which extend the ends of the air supply pipes (see Fig. 1). Magnets with negative polarization (42) are attached to the lower side of the lid (41) located in the second pressure relief chamber.

The dimensions of the upper part of the shaft, or the air intake chamber (2), are larger (this is necessary in order for the air speed to be as low as possible in the air intake chamber as compared to the air speed in the rest of the system) than the dimensions of the first pressure relief chamber, so that on the transition between the first pressure relief chamber (3) and the air intake chamber (2) a step (30) is formed onto which a lid (31) is arranged and secured to separate the first pressure relief chamber (3) from the air intake chamber (2) together with the third pressure relief chamber (5) and the air distribution chamber (7) extending through it to the first pressure relief chamber (3). The air intake chamber (2) is closed at the top with a shaft lid (20). If necessary, the air intake chamber (2) is covered with insulation from the outside to provide a more even temperature in the air intake chamber (2) when the outside temperature drops, for example, below O°C degrees. There are air intake openings (21) provided on the sides of the air intake chamber, located, for example, on the perimeter of the upper edge of the air intake chamber. The air intake openings (21) are provided with cleaning filters (22). Cleaning filters (22) are intended for the primary cleaning of the ambient air; filters remove the larger dust particles, insects, debris raised by the wind, as well as the larger fungal spores and the like from the incoming ambient air. This so-called rough cleaning of the incoming air takes place in the filters. There are also openings on the walls of the air intake chamber (2) for an air pipeline going from the building to the purification system and for an air pipeline going from the purification system to the building.

The air intake chamber (2) is connected to the first pressure relief chamber (3) through the primary ambient air heat exchange pipes (32; pipeline). The primary heat exchange pipes (32) starts from the bottom of the air intake chamber (from the horizontal step (30) formed by the shaft on the transition point between the air intake chamber of the shaft (2) and the first pressure relief chamber (3)), and from there the pipes are directed to the outside of the shaft into the natural ground and reach through the wall of the middle part of the shaft into the first pressure relief chamber. Like this, the earth geothermal energy is utilized for the primary heating of the inlet ambient air and to increase the negative charge of the airborne negative air ions.

The third pressure relief chamber (5) is attached to the lid (31) closing the first pressure relief chamber. The air distribution chamber (7), with cylindrical housing, is passing through the third pressure relief chamber (5) and is connected to the air outlet pipe (70, pipeline) going from the building to the system. Ventilation pipe (8, pipeline), through which the air enriched with positive ions together with pollutants (dust, pathogens, fungal spores, etc.) is guided out of the system, passes through air distribution chamber (7) upward. The third pressure relief chamber 5 is connected to the discharge pipe (9, pipeline) through which the air purified in the system and enriched with negative air ions is directed into the building. Air inlet pipes (10) beginning from the second pressure relief chamber reach the third pressure relief chamber.

The ion purification central unit is resting on the lid (41) at the bottom of the first pressure relief chamber (3) and comprises an ion purification device (11) made of perforated material (e.g. metal) and filled with ceramsite (or the like, ionic material like expanded clay) along with a ventilation pipe (8) for discharging the polluted air from the system. Also, positive air ions that are unhealthy, are also taken out through the ventilation pipe (8). The lower portion of the ion purification device (81) is conical and form a ventilation chamber (82), which contains the lower, and also cone-shaped, portion (80) of the ventilation pipe (8). The ion purification device (11) is partially surrounded by the air distribution chamber (7), so as the air entering the air distribution chamber from the building moves downward in the air distribution chamber (7), and the positively charged ions of the air, along with the pollution and air humidity, are attracted towards the positively charged ceramsite, pass through it and travel towards the inlet end of the ventilation pipe, where they are pulled into the ventilation pipe and taken along the ventilation pipe out of the system.

The natural ambient air (in urban conditions, it is contaminated with dust particles, gases, etc.) enters the ion purification geothermal device through the ambient air intake openings at the upper edge of the air intake chamber (2) on the ground. The primary (rough) cleaning filters (22) for catching the larger dust particles and soot particles, insects, rubbish, and the like flowing in the ambient air, and for the primary cleaning of ambient air entering the system are arranged in the air intake openings. The ambient air is driven from the air intake chamber (2) through the ambient air primary heat exchange pipes into the first pressure relief chamber (3). The ambient air meets the return air from the building in the first pressure chamber (3), so that the ambient air does not mix with the return air from the building, whereas the positive ions are predominant in the return air of the building, bringing along dust and other particles, as well as water vapor from the air of the building. In the upper part of the first pressure relief chamber (3), a cylindrical air separation chamber (7) extends, into which enters the discharge pipe (70) of the air coming from the building. In addition, an ion purification device (11) made of perforated metal and filled with ceramsite, is arranged in the first pressure chamber (3).

The air separation chamber (7) is partially surrounding the ion purification device (11), i.e. the ion purification device (11) is partially located inside the air separation chamber (7). The ceramsite of the ion purification device (11) is originally a material with a neutral charge, but due to the magnets located in the lower part of the ion purification device, ceramsite is beforehand provided with a positive charge, which causes it to attract positive ions from the air of the building and from the ambient air. As a result, the air supersaturated with positive ions passes through the ceramsite and moves along the ventilation pipe (8) with the pollution and water vapor out of the system. Part of the outside air is drawn by ceramsite towards ceramsite, which means, that positively charged air particles move through ceramsite of the ion purification device and thus are taken out of the system through ventilation pile (8). The ambient air directed to the first pressure relief chamber (3) does not mix with air coming from the building and directed to the same first pressure chamber (3) due to the temperature difference. There arises a so-called conflict of temperatures, where the cold air does not mix with the warmer air coming from the building.

An important factor here is the speed of the ambient air and the speed of the air coming from the building. The fresh air intake speed is in the range of 2-6 m/s and the speed of the air blown out of the building is in the range of 3-7 m/s.

There is an underpressure in the first pressure relief chamber (3), while the air distribution chamber (7) for the air departing from the building is in an overpressure, i.e. the air distribution chamber is designed in such a way that an additional underpressure is generated in the first pressure relief chamber (3). The entire structure of the first pressure relief chamber (3) is such that positively charged air ions always travel back to the atmosphere through the ventilation pipe (8), with an air entry speed in the range of 0.2-0.5 m/s and exit speed in the range of 3-6 m/s. This prevents the energy accumulation processes (device freezing or overheating).

The air enriched with negative air ions moves from the first pressure chamber (3) to the heat exchange pipes (6), in which the air speed is in the range of 0.5-12 m/s.

With shorter pipes, the speed is lower, while with longer pipes the speed is higher. The air charged with negative ions and heated by the earth's thermal energy moves from the heat exchange pipes (6) to the second pressure relief chamber (4). The second pressure relief chamber (4) is separated from the first pressure relief chamber in an airtight way by a plate (41) made of nonconductive material. The second pressure relief chamber is located in the lowest part of the shaft, with a constantly uniform ambient temperature of approximately + 7-8 °C. There is a filter device (12) filled with negatively charged activated carbon installed in the second pressure relief chamber (4) through which the air charged with negative ions flows into an inlet pipeline (10), which is connected to the third pressure relief chamber (5) located at the top of the system. The follow-up ion purification and filtration of the air takes place in the filter (12) filled with activated carbon. Air velocity is as follows: the air speed inside the second pressure relief chamber (4) is in the range of 0.2-0.5 m/s and the exit speed is in the range of 0.5-3.0 m/s. From the air inlet pipeline (10), the air moves to the third pressure relief chamber (5), with an air speed inside the chamber ranging from 0.3 to 1.2 m/s, and the speed of the air blown out of the chamber ranging from 2-6 m/s. The outlet pipe (9) of the air drawn into the building is connected to the third pressure relief chamber (5) and the ion-purified air moves, for example, to the heat recovery ventilation unit of the building (not shown), whereas the air is ion-purified or "fresh" (saturated with negative ions and also preheated). The temperature of the air entering the building is in winter, for example, always +1-8 °C and in summer always +16-21 °C.

All the air is treated in the system with natural ion exchange without use of additional energy. In the structure according to the invention, this is done in an underground ion purification device (11) where positive and negative aeroions and the opposite temperatures meet (the air exiting the building and the outside air meet in the first pressure relief chamber (3)) and where air ion-purification and temperature changes always take place (warming during the cold season or cooling during the warm season, respectively), whereas negative ions are used to create in the ambient air or in the air coming from the building an environment which inhibits the life processes of pathogens (bacteria, viruses, insects, etc.),. The life processes of bacteria that degrade the living environment and of viruses are essentially decreased or stopped.

Advantages of the system according to the invention are as follows:
- the air blown into the building does not release any natural moisture
- the indoor air of the buildings lacks active dust (i.e. is missing flying dust), that is, the indoor air of the buildings is virtually dust-free, because natural air humidity is used to turn the dust particles into passive dust (passive dust does not fly)
- earth's thermal energy is used to reduce the costs of heating the air that is drawn in (especially during the cold period (winter)) for approximately 60% (using heat recovery ventilation systems (especially in winter), heating costs can be reduced by about 98%)
- the temperature of the air blown into the building during summer is between +21-28 °C (in case of heat recovery ventilation systems, summer mode is used in summer),
- the saving on ventilation maintenance costs is of about 80%, since, together with positive air ions, dust particles, bacteria and the like are removed from the system, meaning that there are no suitable reproduction and living conditions for pathogens,
- spreading of mold stops, the system and the building develop no moisture damages,
- the radon released from the earth does not penetrate into the building, but is also removed from the system through the ventilation pipeline,
- people's health will improve, because the so-called fresh air contains to a large extent negative air ions,
- finally, the higher the difference between the temperature of the atmosphere and the internal constant temperature of the earth, the higher is the efficiency of the ion purification system according to the invention.

### List of details:

1 - housing, shaft
2 - air intake chamber
20 - shaft lid, closes the air intake chamber
21 - ambient air intake openings
22 - cleaning filters
3 - first pressure relief chamber
30 - step on the transition of the air intake chamber and first pressure relief chamber, the step is horizontal
31 - lid, separates the air intake chamber from the first pressure relief chamber
32 - ambient air primary inlet pipes
4 - second pressure relief chamber
41 - lid, separates the first pressure relief chamber from the second pressure relief chamber
42 - magnets with a negative polarization
5 - third pressure relief chamber
6 - heat exchange pipes
7 - air distribution chamber
70 - outlet pipe for the air directed from the building to the system
8 - ventilation pipe
82 - ventilation chamber
9 - outlet pipe
10 - air inlet pipe
11 - ion purification device
12 - filter device, filled with activated charcoal

## Claims

1. Geothermal ion purification device for a building, comprising a vertical housing/shaft (1) fully or partially placeable into the ground and made up of three parts: an upper part or air intake chamber (2), a middle part or first pressure relief chamber (3) and a lower part or second pressure relief chamber (4), wherein ambient air primary heat exchange pipes (32) are connecting the air intake chamber (2) with the first pressure relief chamber (3), wherein heat exchange pipes (6) are connecting the first pressure relief chamber (3) with the second pressure relief chamber (4), wherein the air intake chamber (2) is closed on the top with a shaft lid (20), wherein ambient air intake openings (21) are located on the perimeter of the upper edge of the sides of the air intake chamber (2), said ambient air intake openings (21) being provided with cleaning filters (22), wherein the dimensions of the upper part of the shaft or air intake chamber (2) are larger than the dimensions of the first pressure relief chamber (3), so that a step (30) is formed on the transition between the first pressure relief chamber (3) and the air intake chamber (2) onto which a lid (31) is placed and secured, said lid (31) separates the first pressure relief chamber (3) from the air intake chamber (2), wherein a third pressure relief chamber (5) is attached to the lid (31), said lid (31) further separates the third pressure relief chamber (5) from the first pressure relief chamber (3), wherein air inlet pipes are connecting the third pressure relief chamber (5) to the second pressure relief chamber (4) and the third pressure relief chamber (5) is connected to an outlet pipe (9) through which the air purified in the device and enriched with negative aeroions is guided to the building, wherein an air distribution chamber (7) is extending through the third pressure relief chamber (5) and the lid (31) to the first pressure relief chamber (3), wherein the air distribution chamber (7) is connected to the outlet pipe (70) for the air directed from the building to the system, wherein the dimensions of the first pressure relief chamber (3) are larger than the dimensions of the second pressure relief chamber (4), so that on the transition between the first and second pressure relief chambers a step (40) has been formed onto which a lid (41) has been placed and secured, said lid (41) separating the second pressure relief chamber (4) from the first pressure relief chamber (3), wherein an ion purification central unit rests on the lid (41) at the bottom of the first pressure relief chamber (3), said ion purification central unit comprising an ion purification device (11) made of perforated material and filled with ceramsite or lightweight expanded clay along with a ventilation pipe (8), provided to remove the polluted air from the system, the lower part of the ion purification device (81) being conical and forming a ventilation chamber (82) containing the lower, and also cone-shaped, end (80) of the ventilation pipe (8), wherein the ends of the air inlet pipes are extending through the lid (41), wherein magnets with negative polarization (42) are attached to the lower side of the lid (41), which is located in the second pressure relief chamber (4), and wherein a filter device (12) filled with negatively charged activated carbon is located in the second pressure relief chamber (4).

2. Geothermal ion purification device according to claim 1, **characterized in that** at least the first pressure relief chamber (3) and the second pressure relief chamber (4) are placeable below the ground level.

3. Geothermal ion purification device according to claim 1 or 2, **characterized in that**, openings are provided in the wall of the air intake chamber (2) for the outlet pipe (70) for the air coming from the building to the purification system and for the outlet pipe (9) for the air moving from the system to the building.

4. Geothermal ion purification device according to any of claims 1 to 3, **characterized in that** the primary heat exchange pipes (32) begin from the bottom of the air intake chamber and from there the pipes are directed outwards of the shaft and adapted to be directed into the natural ground and reach through the wall of the middle part of the shaft to the first pressure relief chamber.

5. Geothermal ion purification device according to any of claims 1 to 4, **characterized in that** the third pressure relief chamber (5) is attached to the lid (31) thereby closing the first pressure relief chamber (3).

6. Geothermal ion purification device according to any of the preceding claims, **characterized in that** the upward ventilation pipe (8) for removing the positive aeroions from the device is extending through the air distribution chamber (7).

7. Geothermal ion purification device according to any of the preceding claims, **characterized in that** the ion purification device (11) is partly surrounded by the air distribution chamber (7), so that the air entering the air distribution chamber from the building moves downwards in the air distribution chamber (7) and the positively charged aeroions of the air together with pollution and atmospheric moisture draw towards the positively charged ceramsite, pass through it and head towards the inlet end of the ventilation pipe where they are drawn into the ventilation pipe and taken out of the device along the ventilation pipe.

8. A method for changing the indoor climate of a building and for purifying the air with a geothermal ion purification device according to claims 1-7 using the geothermal energy and negative potential of the earth, which comprises steps in which:
- the ambient air at a speed of 2-3 m/s is first directed through the air intake openings (21) and ambient air cleaning filters (22) into the air intake chamber (2) after which the ambient air at a speed of 3-6 m/s is guided through the ambient air primary heat exchange pipe (32) to the first pressure relief chamber (3) where
- positively charged air ions with polluted air are drawn into the positively charged material of the ion purification device (11) from which the air is pulled through and guided through the ventilation pipe (8) out of the system at a speed of 3-7 m/s into the surrounding atmosphere,
- negatively charged air ions together with the air are drawn at a speed of 0.2-0.5 m/s from the first pressure relief chamber (3) through the heat exchange pipes (6) to the second pressure relief chamber (4), whereas the air is moving in the heat exchange pipes at a speed of 0.5-1.2 m/s, during which the air moving in the heat exchange tubes is heated with the geothermal energy of the earth,
- an additional negative charge is supplied to the heated air by the negative pole magnet (42) mounted to the lid (41) of the second pressure relief chamber (4) and the air is driven at a speed of 0.5-3.0 m/s through an activated carbon filled filter device (12) and flows into air inlet pipes to the third pressure relief chamber (5),
- the air that is purified and enriched with negative ions is driven from the third pressure relief chamber (5) through the outlet pipe (9) at a speed of 3-6 m/s.

## Patentansprüche

1. Geothermische lonenreinigungsvorrichtung für Gebäude, mit einem vertikalen Gehäuse / Schacht (1), das ganz oder teilweise in den Boden eingelassen werden kann und aus drei Teilen besteht: einem oberen Teil oder einer Lufteinlasskammer (2), einem mittleren Teil oder der ersten Druckentlastungskammer (3) und einem unteren Teil oder der zweiten Druckentlastungskammer (4), wobei Rohre für den Primärwärmetausch der Umgebungsluft (32) die Luftansaugkammer (2) mit der ersten Druckentlastungskammer (3) verbinden, wobei Wärmetauscherrohre (6) die erste Druckentlastungskammer (3) mit der zweiten Druckentlastungskammer (4) verbinden, wobei die Luftansaugkammer (2) oben mit einem Schachtdeckel (20) verschlossen ist, wobei Umgebungsluftansaugöffnungen (21) am Umfang des oberen Randes der Seiten der Luftansaugkammer angeordnet sind (2), wobei die Umgebungsluftansaugöffnungen (21) mit Reinigungsfiltern (22) ausgestattet sind, wobei die Abmessungen des oberen Teils des Schachtes oder der Luftansaugkammer (2) größer sind als die Abmessungen der ersten Druckentlastungskammer (3), so dass am Übergang zwischen der ersten Druckentlastungskammer (3) und der Luftansaugkammer (2) eine Stufe (30) vorhanden ist, auf der ein Deckel (31) aufgesetzt und befestigt ist, der die erste Druckentlastungskammer (3) von der Luftansaugkammer (2) trennt, wobei der Deckel (31) ferner die dritte Druckentlastungskammer (5) von der ersten Druckentlastungskammer (3) trennt, wobei Lufteinlassrohre (10) die dritte Druckentlastungskammer (5) mit der zweiten Druckentlastungskammer (4) und die dritte Druckentlastungskammer (5) mit einem Auslassrohr (9) verbunden ist, wodurch die in der Vorrichtung gereinigte und mit negativen Aeroionen angereicherte Luft in das Gebäude geleitet wird, wobei die Luftverteilungskammer (7) durch die dritte Druckentlastungskammer (5) und den Deckel (31) zur ersten Druckentlastungskammer (3) führt, wobei die Luftverteilungskammer (7) mit dem Auslassrohr (70) für die vom Gebäude zum System geleitete Luft verbunden ist, wobei die Abmessungen der ersten Druckentlastungskammer (3) größer sind als die Abmessungen der zweiten Druckentlastungskammer (4), so dass am Übergang zwischen der ersten und der zweiten Druckentlastungskammer eine Stufe (40) vorhanden ist, auf die ein Deckel (41) aufgesetzt und befestigt ist, der die zweite Druckentlastungskammer (4) von der ersten Druckentlastungskammer (3) trennt, wobei auf dem Deckel (41) am Boden der ersten Druckentlastungskammer (3) die zentrale lonenreinigungseinheit angeordnet ist, die eine lonenreinigungsvorrichtung (11) aus perforiertem Material umfasst, die mit Keramsit oder Leichtblähton gefüllt ist, sowie ein Belüftungsrohr (8), wobei der untere Teil der lonenreinigungsvorrichtung (81) konisch ist und eine Entlüftungskammer (82) bildet, die das untere, ebenfalls konische Ende (80) des Entlüftungsrohrs (8) enthält, wobei die Enden der Luftzuführungsrohre (10) durch den Deckel (41) hindurchgeführt sind, wobei an der Unterseite des Deckels (41), der sich in der zweiten Druckentlastungskammer (4) befindet, Magnete mit negativer Polarisation (42) befestigt sind, und wobei sich in der zweiten Druckentlastungskammer (4) eine mit negativ geladener Aktivkohle gefüllte Filtereinrichtung (12) befindet.

2. Geothermische lonenreinigungsvorrichtung nach Anspruch 1, **gekennzeichnet dadurch, dass** zumindest die erste Druckentlastungskammer (3) und die zweite Druckentlastungskammer (4) unterhalb des Bodenniveaus angeordnet werden können.

3. Geothermische lonenreinigungsvorrichtung nach Anspruch 1 oder 2, **gekennzeichnet dadurch, dass** in der Wand der Lufteinlasskammer (2) Öffnungen für das Auslassrohr (70) für die vom Gebäude zum Reinigungssystem kommende Luft und für das Auslassrohr (9) der vom System zum Gebäude gehenden Luft vorgesehen sind.

4. Geothermische lonenreinigungsvorrichtung nach einem der Ansprüche 1 bis 3, **gekennzeichnet dadurch, dass** die Rohre für den Primärwärmetausch (32) am Boden der Lufteintrittskammer beginnen und von dort aus dem Schacht herausgeführt sind und durch die Wand des Mittelteils des Schachtes bis zur ersten Druckentlastungskammer reichen.

5. Geothermische lonenreinigungsvorrichtung nach einem der Ansprüche 1 bis 4, **gekennzeichnet dadurch, dass** die dritte Druckentlastungskammer (5) an dem Deckel (31) befestigt ist und dadurch die erste Druckentlastungskammer (3) verschließt.

6. Geothermische lonenreinigungsvorrichtung nach einem der vorhergehenden Ansprüche, **gekennzeichnet dadurch, dass** das nach oben gerichtete Belüftungsrohr (8) zum Entfernen der positiven Aeroionen aus der Vorrichtung durch die Luftverteilungskammer (7) verläuft.

7. Geothermische lonenreinigungsvorrichtung nach einem der vorhergehenden Ansprüche, **gekennzeichnet dadurch, dass** die lonenreinigungsvorrichtung (11) teilweise von der Luftverteilungskammer (7) umgeben ist, so dass die vom Gebäude in die Luftverteilungskammer einströmende Luft sich in der Luftverteilungskammer (7) nach unten bewegt und die positiv geladenen Luftaerionen zusammen mit Verunreinigungen und atmosphärischer Feuchtigkeit zu dem positiv geladenen Keramsit strömen, es durchqueren und zum Einlassende des Belüftungsrohrs gelangen, wo sie in das Belüftungsrohr gezogen und entlang des Belüftungsrohrs aus der Vorrichtung herausgeführt werden.

8. Verfahren zur Beeinflussung des Innenraumklimas in einem Gebäude und zur Luftreinigung mit einem geothermischen lonenreinigungsgerät nach einem der Ansprüche 1-7 unter Nutzung der geothermischen Energie und des negativen Potentials der Erde, das die Schritte umfasst, bei denen
- die Umgebungsluft mit einer Geschwindigkeit von 2-3 m/s zunächst durch die Lufteintrittsöffnungen (21) und Umgebungsluftreinigungsfilter (22) in die Lufteintrittskammer (2) geleitet wird, wonach die Umgebungsluft mit einer Geschwindigkeit von 3-6 m/s durch das Rohr für den Primärwärmetausch der Umgebungsluft (32) in die erste Druckentlastungskammer (3) geleitet wird, wo
- positiv geladene Luftionen mit verunreinigter Luft in das positiv geladene Material der lonenreinigungsvorrichtung (11) eingezogen werden, aus dem die Luft herausgezogen und durch das Belüftungsrohr (8) mit einer Geschwindigkeit von 3-7 m/s aus dem System in die umgebende Atmosphäre geleitet wird,
- negativ geladene Luftionen zusammen mit der Luft mit einer Geschwindigkeit von 0,2 - 0,5 m/s aus der ersten Druckentlastungskammer (3) durch die Wärmetauscherrohre (6) in die zweite Druckentlastungskammer (4) gezogen werden, während die Luft in den Wärmetauscherrohren mit einer Geschwindigkeit von 0,5 - 1,2 m/s strömt, wobei die in den Wärmetauscherrohren strömende Luft mit der geothermischen Energie der Erde erhitzt wird,
- der erwärmten Luft durch den am Deckel (41) der zweiten Druckentlastungskammer (4) angebrachten Minuspolmagneten (42) eine zusätzliche negative Ladung zugeführt wird und die Luft mit einer Geschwindigkeit von 0,5-3,0 m/s durch eine mit Aktivkohle gefüllte Filtereinrichtung (12) geleitet wird und in Lufteinlassleitungen (10) zur dritten Druckentlastungskammer (5) strömt,
- die gereinigte und mit negativen Ionen angereicherte Luft mit einer Geschwindigkeit von 3-6 m/s aus der dritten Druckentlastungskammer (5) durch das Auslassrohr (9) geleitet wird.

## Revendications

1. Dispositif géothermique de purification d'ions pour bâtiment, comprenant un boîtier/puits vertical (1) entièrement ou partiellement encastrable dans le sol et constitué de trois parties : une partie supérieure ou chambre d'admission d'air (2), une partie médiane ou première chambre de décompression (3), et une partie inférieure ou deuxième chambre de décompression (4), dans laquelle des tuyaux primaires d'échange thermique (32) de l'air ambiant relient la chambre d'admission d'air (2) à la première chambre de décompression (3), dans laquelle des tuyaux d'échange thermique (6) relient la première chambre de décompression (3) à la deuxième chambre de décompression (4), dans laquelle la chambre d'admission d'air (2) est fermée sur le dessus par un couvercle de puits (20), dans lequel des ouvertures d'admission d'air ambiant (21 ) sont situés sur le périmètre du bord supérieur des côtés de la chambre d'admission d'air (2), lesdites ouvertures d'admission d'air ambiant (21) étant munies de filtres de purification (22), dans lequel les dimensions de la partie supérieure du puits ou de la chambre d'admission d'air (2) sont plus importantes que les dimensions de la première chambre de décompression (3), de sorte qu'une marche (30) est formée sur la transition entre la première chambre de décompression (3) et la chambre d'admission d'air (2) sur laquelle un couvercle (31) est placé et fixé, ledit couvercle (31) séparant la première chambre de décompression (3) de la chambre d'admission d'air (2), dans laquelle une troisième chambre de décompression (5) est fixée au couvercle (31), ledit couvercle (31) séparant en outre la troisième chambre de décompression (5) de la première chambre de décompression (3), dans laquelle des tuyaux d'entrée d'air (10) relient la troisième chambre de décompression (5) à la deuxième chambre de décompression(4), et la troisième chambre de décompression (5) est reliée à un tuyau de sortie (9) à travers lequel l'air purifié dans le dispositif et enrichi en aéroions négatifs est conduit vers le bâtiment, dans lequel une chambre de distribution d'air (7) s'étend à travers la troisième chambre de décompression (5) et le couvercle (31) jusqu'à la première chambre de décompression (3), dans laquelle la chambre de distribution d'air (7) est reliée au tuyau de sortie (70) pour l'air conduit du bâtiment au système, dans lequel les dimensions de la première chambre de décompression (3) sont plus importantes que les dimensions de la deuxième chambre de décompression (4), de sorte qu'une marche (40) est formée sur la transition entre la première et la deuxième chambre de décompression, sur laquelle un couvercle (41) est placé et fixé, ledit couvercle (41) séparant la deuxième chambre de décompression (4) de la première chambre de décompression (3), dans laquelle une unité centrale de purification d'ions repose sur le couvercle ( 41) au fond de la première chambre de décompression (3), ladite unité centrale de purification d'ions comprenant un dispositif de purification d'ions (11) fait de matériau perforé et rempli de céramsite ou d'argile expansée ultralégère, ainsi qu'un tuyau de ventilation (8), prévu pour éliminer l'air pollué du système, la partie inférieure du dispositif de purification d'ions (81) étant conique et formant une chambre de ventilation (82) contenant l'extrémité (80) inférieure, également conique, du tuyau de ventilation (8), dans lequel les extrémités des tuyaux d'entrée d'air (10) s'étendent à travers le couvercle (41), dans lequel des aimants à polarisation négative (42) sont fixés sur le côté inférieur du couvercle (41), qui est situé dans la deuxième chambre de décompression (4), et dans lequel un dispositif de filtre (12) rempli de charbon actif au chargement négatif est situé dans la deuxième chambre de décompression (4).

2. Dispositif géothermique de purification d'ions selon la revendication 1, **caractérisé en ce qu'**au moins la première chambre de décompression (3) et la deuxième chambre de décompression (4) sont encastrables en dessous du niveau du sol.

3. Dispositif géothermique de purification d'ions selon la revendication 1 ou 2, **caractérisé en ce que** des ouvertures sont prévues dans la paroi de la chambre d'admission d'air (2) pour le tuyau de sortie (70) de l'air conduit du bâtiment au système de purification et pour le tuyau de sortie (9) de l'air circulant du système vers le bâtiment.

4. Dispositif géothermique de purification d'ions selon l'une quelconque des revendications 1 à 3, **caractérisé en ce que** les tuyaux primaires d'échange thermique (32) partent du fond de la chambre d'admission d'air et de là les tuyaux sont dirigés vers l'extérieur du puits et adaptés pour être dirigés dans le sol naturel et pour atteindre la paroi de la partie médiane du puits jusqu'à la première chambre de décompression.

5. Dispositif géothermique de purification d'ions selon l'une quelconque des revendications 1 à 4, **caractérisé en ce que** la troisième chambre de décompression (5) est fixée au couvercle (31), fermant ainsi la première chambre de décompression (3).

6. Dispositif géothermique de purification d'ions selon l'une quelconque des revendications précédentes, **caractérisé en ce que** le tuyau de ventilation (8) ascendant pour évacuer les aéroions positifs du dispositif s'étend à travers la chambre de distribution d'air (7).

7. Dispositif géothermique de purification d'ions selon l'une quelconque des revendications précédentes, **caractérisé en ce que** le dispositif de purification d'ions (11) est en partie entouré par la chambre de distribution d'air (7), de sorte que l'air entrant dans la chambre de distribution d'air depuis le bâtiment circule vers le bas dans la chambre de distribution d'air (7) et les aéroions de l'air au chargement positif ainsi que la pollution et l'humidité atmosphérique se dirigent vers la céramsite au chargement positif, la traversent et se dirigent vers l'extrémité d'entrée du tuyau de ventilation où ils sont aspirés dans le tuyau de ventilation et évacués du dispositif le long du tuyau de ventilation.

8. Procédé pour modifier le climat intérieur d'un bâtiment et pour purifier l'air au moyen d'un dispositif géothermique de purification d'ions selon les revendications 1 à 7 utilisant l'énergie géothermique ainsi que le potentiel négatif de la terre, comprenant les étapes dans lesquelles :
- l'air ambiant circulant à une vitesse de 2 à 3 m/s est d'abord dirigé à travers les ouvertures d'admission d'air (21) et les filtres de purification (22) de l'air ambiant dans la chambre d'admission d'air (2), après quoi l'air ambiant circulant à une vitesse de 3 à 6 m/s est guidé à travers le tuyau primaire d'échange thermique (32) de l'air ambiant dans la première chambre de décompression (3) où
- les ions d'air au chargement positif avec l'air pollué sont aspirés dans le matériau au chargement positif du dispositif de purification d'ions (11) à partir duquel l'air est aspiré et guidé à travers le tuyau de ventilation (8) hors du système, à une vitesse de 3 à 7 m/s, dans l'atmosphère environnante,
- les ions d'air au chargement négatif avec l'air sont aspirés à une vitesse de 0,2 à 0,5 m/s depuis la première chambre de décompression (3) à travers les tuyaux d'échange thermique (6) dans la deuxième chambre de décompression (4), tandis que l'air circule à l'intérieur des tuyaux d'échange thermique à une vitesse de 0,5 à 1,2 m/s, pendant laquelle l'air circulant à l'intérieur des tuyaux d'échange thermique est chauffé au moyen de l'énergie géothermique de la terre,
- un chargement négatif supplémentaire est fournie à l'air chauffé par l'aimant à pôle négatif (42) monté sur le couvercle (41) de la deuxième chambre de décompression (4), et l'air est entraîné à une vitesse de 0,5 à 3,0 m/s à travers un dispositif de filtre (12) rempli de charbon actif et circule dans les tuyaux d'entrée d'air (10) jusqu'à la troisième chambre de décompression (5),
- l'air qui est purifié et enrichi en ions négatifs est évacué de la troisième chambre de décompression (5) à travers le tuyau de sortie (9) à une vitesse de 3 à 6 m/s.
